# EUROPÄISCHE PATENTSCHRIFT

(11) **EP 1 703 929 B1**
(45) Veröffentlichungstag und Bekanntmachung des Hinweises auf die Patenterteilung: **21.10.2009**
(21) Anmeldenummer: 04797272.4
(22) Anmeldetag: 29.11.2004
(51) Int. Cl.: A61M 5/20, A61M 5/32

(54) **AUSLÖSBARES INJEKTIONSGERÄT**
RELEASABLE INJECTION DEVICE
DISPOSITIF D'INJECTION DECLENCHABLE

(30) Priorität: 18.12.2003 CH 218903; 18.12.2003 DE 20319648 U
(43) Veröffentlichungstag der Anmeldung: 27.09.2006
(73) Patentinhaber: TecPharma Licensing AG, 3401 Burgdorf (CH)
(72) Erfinder: HOMMANN, Edgar, CH-3257 GROSSHOECHSTETTEN (CH)
(86) Internationale Anmeldenummer: PCT/CH2004/000715
(87) Internationale Veröffentlichungsnummer: WO 2005/058396

(56) Entgegenhaltungen:
- EP-A- 0 824 923
- DE-A- 4 037 418
- GB-A- 1 132 065
- US-A- 3 563 098
- US-A- 5 599 309

## Beschreibung

Die Erfindung betrifft ein Injektionsgerät für die Verabreichung eines injizierbaren Produkts, vorzugsweise eines flüssigen Medikaments wie beispielsweise Insulin, ein Wachstumshormon, Heparin oder ein Osteoporosepräparat. Das Injektionsgerät ist vorzugsweise ein Injektionspen oder ein Autoinjektor.

Injektionsgeräte sind in vielerlei Ausführungen bekannt. Sie dienen insbesondere zur Verabreichung von Medikamenten, die sich der betroffene Patient selbst injiziert. Viele der bekannten Autoinjektoren weisen eine Auslösesicherung auf, die verhindern soll, dass unbeabsichtigt ein Injektionsvorgang ausgelöst wird. Ein Beispiel für einen Autoinjektor ist im Dokument DE4037418 beschrieben. Dieser Autoinjektor weist ein Gehäuse zur Aufnahme eines Wirkstoffbehälters auf, wobei das Gehäuse aus zwei Teilen besteht, die entlang einer Achse zwischen einer ersten und einer zweiten Stellung relativ zueinander bewegbar sind. Ferner weist der Autoinjektor einen Freigabemechanismus, eine durch den Freigabemechanismus in einer durch eine gespannte Feder belasteten Lage gehaltene Ausgabeeinrichtung zum Ausgeben des Wirkstoffs und eine Auslöservorrichtung in der Form eines Druckknopfes auf, welcher ebenfalls in Richtung der genannten Achse betätigbar ist. Dabei weist das eine Gehäuseteil die Auslöservorrichtung und das andere Gehäuseteil den Freigabemechanismus auf. In der ersten Stellung der beiden Gehäuseteile relativ zueinander ist der Freigabemechanismus weiter von der der Auslöservorrichtung entfernt als in der zweiten Stellung und eine Betätigung der Auslösevorrichtung bleibt wirkungslos. In der zweiten Stellung der Gehäuseteile ist die Auslösevorrichtung näher am Freigabemechanismus und daher fähig, diesen zu veranlassen, die Ausgabeeinrichtung freizugeben.

Ausgehend von diesem Stand der Technik liegt der Erfindung die Aufgabe zugrunde, ein Injektionsgerät vorzuschlagen, das mit nur wenigen Bestandteilen eine gute Sicherheit gegen unbeabsichtigtes Auslösen bietet.

Diese Aufgabe wird gemäss der vorliegenden Erfindung durch einen Autoinjektor nach Anspruch 1 und ein auslösbares Injektionsgerät nach Anspruch 11 gelöst.

In einer Ausführungsform der Erfindung umfasst das Injektionsgerät einen Gehäuseabschnitt, ein Reservoir für das zu injizierendes Produkt, nämlich den Wirkstoff, eine Injektionsnadel, einen Nadelschutz, eine Antriebseinrichtung für die Injektion, und eine Auslösemechanik für die Auslösung der Antriebseinrichtung. Der Gehäuseabschnitt oder ein damit verbundener weiterer Gehäuseabschnitt des Injektionsgeräts können das Reservoir unmittelbar bilden. Vorzugsweise ist das Reservoir jedoch ein Behältnis, das in dem Gehäuseabschnitt oder dem weiteren Gehäuseabschnitt aufgenommen ist. Vorzugsweise umfasst das Injektionsgerät einen Reservoirhalter, der das Reservoir hält und zentriert und selbst in einem Gehäuseabschnitt gehalten und zentriert ist. Die Injektionsnadel kann insbesondere an dem Reservoir oder grundsätzlich auch an dem Reservoirhalter befestigt sein. Sie ragt dementsprechend axial vorzugsweise von dem Reservoir oder dem Reservoirhalter vor mit einer Nadelspitze an ihrem vorderen, d.h. distalen Ende. Der Nadelschutz ist mit dem Gehäuseabschnitt so verbunden, dass er relativ zu dem Gehäuseabschnitt und insbesondere zu der Injektionsnadel aus einer Schutzposition, in der er die Injektionsnadel bis über deren Nadelspitze umgibt, nach hinten, d.h. proximal, vorzugsweise axial, relativ zu dem Gehäuseabschnitt und der Injektionsnadel bis in eine Rückzugsposition bewegbar ist. Der Nadelschutz kann ein reiner Sichtschutz sein, der gegen eine elastische Rückstellkraft nach proximal bewegt werden kann und bei Entlastung aufgrund der Rückstellkraft wieder in die distale Richtung vorschiebt. Er kann in der Schutzposition aber alternativ auch gegen eine Rückzugsbewegung blockiert sein, wobei die Blockierung vor der Injektion lösbar sein muss und danach lösbar oder unlösbar sein kann.

Die Antriebseinrichtung kann aus einem Ausschüttantrieb bestehen, der ein Förderelement oder Treibelement und einen Ausschüttantrieb für das Förderelement aufweist, wobei das Förderelement für die Produktausschüttung auf das in dem Reservoir befindliche Produkt wirkt. In bevorzugten Ausführungen, in denen das Injektionsgerät ein Autoinjektor ist, umfasst die Antriebsvorrichtung nicht nur solch einen Ausschüttantrieb, sondern zusätzlich auch einen Einstechantrieb für die Injektionsnadel. Dabei kann der gleiche Krafterzeuger sowohl den Ausschüttantrieb als auch den Einstechantrieb bilden. So kann insbesondere eine mechanische Feder, vorzugsweise eine auf Druck vorgespannte Spiralfeder, den Einstechantrieb und auch den Ausschüttantrieb bilden. Für jeden der beiden Antriebe können jedoch auch separate Krafterzeuger, beispielsweise je eine auf Druck vorgespannte Spiralfeder, verwendet werden. Vorteilhafterweise ist insbesondere der Einstechantrieb ein Krafterzeuger, der vor einer Injektion einen elastisch gespannten Zustand einnimmt, aus dem heraus er die Injektionsnadel zum Einstechen in oder vorzugsweise durch die Haut nach distal, d.h. in eine Vortriebsrichtung, treibt. Die Antriebseinrichtung kann so ausgeführt sein, dass sie nur eine die Ausschüttung bewirkende Antriebsbewegung oder eine die Ausschüttung und ein Vorstechen bewirkende einheitliche Antriebsbewegung ausführt, oder dass sie eine Antriebsbewegung für das Vorstechen und eine weitere Antriebsbewegung für das Ausschütten ausführt.

In einem Ausgangszustand des Injektionsgeräts ist die Antriebseinrichtung in einem lösbaren Halteeingriff gehalten, so dass sie keine Antriebsbewegung, insbesondere keine das Vorstechen der Injektionsnadel bewirkende Antriebsbewegung ausführen kann. Handelt es sich bei dem Injektionsgerät nicht um einen Autoinjektor, sondern um einen Injektor, dessen Injektionsnadel ein Verwender selbst einstechen muss, so besteht der Halteeingriff dementsprechend nur mit dem in solch einem Fall nur vorhandenen Ausschüttantrieb.

Für die Auslösung der Antriebseinrichtung wirken ein beweglicher Gehäuseabschnitt oder der Nadelschutz, das Auslöseglied oder Auslöseelement und ein Freigabeelement, insbesondere ein Schaltglied zusammen. In dieser Ausführungsform sind Schaltglied und der Nadelschutz so miteinander gekoppelt, dass die Bewegung des Nadelschutzes aus der Schutzposition in die Rückzugsposition eine Bewegung des Schaltglieds aus einer Verriegelungs- bzw. Sperrposition in eine Koppel- bzw. Zwischenposition bewirkt. Nimmt das Schaltglied die Koppelposition ein, kann durch eine Betätigung des Auslöseglieds das Auslöseglied in einen Koppeleingriff mit dem Schaltglied gebracht werden. In dem Koppeleingriff wird die durch die Betätigung bewirkte Auslösebewegung des Auslöseglieds in eine Bewegung des Schaltglieds aus der Koppelposition in eine Freigabeposition umgewandelt. So lange das Schaltglied die Freigabeposition nicht einnimmt, verhindert es, dass sich der Halteeingriff der Antriebseinrichtung lösen kann. Der Halteeingriff besteht direkt oder über ein oder mehrere Zwischenglieder zwischen der Antriebseinrichtung und dem Gehäuseabschnitt, so dass die Antriebseinrichtung in dem Halteeingriff relativ zu dem Gehäuseabschnitt keine Antriebsbewegung ausführen kann. Die Antriebsbewegung ist oder umfasst vorzugsweise eine axiale Linearbewegung der Antriebseinrichtung in eine Vortriebsrichtung, vorzugsweise nach distal. Ist das Injektionsgerät ein Autoinjektor, so ist die Antriebseinrichtung mit der Injektionsnadel vorzugsweise so gekoppelt, dass sie die Injektionsnadel in die Antriebsrichtung, d.h. in die Richtung ihrer eigenen Antriebsbewegung, treibt.

Das Schaltglied führt die Bewegung aus der Verriegelungsposition über die Koppelposition bis in die Freigabeposition als eine Einheit aus. Hierdurch wird die Zahl der für die Auslösung benötigten Teile gering gehalten. Das Schaltglied ist vorzugsweise einstückig geformt, kann grundsätzlich aber auch als eine zusammengesetzte Struktur gebildet sein, die jedoch die Bewegung aus der Verriegelungsposition bis in die Freigabeposition als Einheit wie ein einziger, zumindest in Bezug auf diese Bewegung steifer Körper ausführt.

Die Bewegung des Schaltglieds von der Verriegelungsposition bis in die Freigabeposition ist vorzugsweise eine Bewegung in nur eine einzige Richtung, vorzugsweise eine Axialbewegung in die proximale Richtung.

Das Auslöseglied ragt vorzugsweise aus dem Gehäuseabschnitt hinaus, so dass es unmittelbar betätigt werden kann. Vorzugsweise ist es als Auslöseknopf, d.h. als Druckknopf, gebildet. Die von dem Auslöseglied im Falle der Betätigung ausgeführte Bewegung ist vorzugsweise eine Bewegung quer zu einer Proximal-Distal-Achse des Injektionsgeräts.

Der Koppeleingriff zwischen dem Auslöseglied und dem Schaltglied wird vorzugsweise durch Gleitdruckkontakt hergestellt. Wenigstens eines aus Schaltglied und Auslöseglied bildet eine Schaltkurve und das andere ein an der Schaltkurve abgleitendes Eingriffsglied. Durch die Auslösebewegung des Auslöseglieds wird der Gleitdruckkontakt hergestellt, wenn das Schaltglied die Koppelposition einnimmt. Im Gleitdruckkontakt drückt das Auslöseglied das Schaltglied aus der Koppelposition in die Freigabeposition.

In den Positionen, die das Schaltglied bei einer Injektion vor Einnahme der Freigabeposition einnimmt, sperrt es eine den Halteeingriff zwischen der Antriebseinrichtung und dem Gehäuseabschnitt bewirkende Blockiereinrichtung gegen ein Lösen des Halteeingriffs. Die Blockiereinrichtung ist elastisch auf ein Lösen des Halteeingriffs gespannt, wird aber an dem Lösen des Eingriffs durch das Schaltglied gehindert. Vorzugsweise drückt das Schaltglied ein Blockierelement der Blockiereinrichtung in den Halteeingriff. Das Schaltglied ist so geformt, dass es in der Freigabeposition zulässt, dass das Blockierelement aus dem Halteingriff schnappt, wodurch der Halteeingriff automatisch gelöst wird.

Nach einer Ausführungsart der Erfindung in Form eines Autoinjektors ist das Auslöseelement bzw. Auslöseglied in einer Ebene quer zur Längsrichtung des Autoinjektors und das Freigabeelement in Längsrichtung des Autoinjektors beweglich. Das Freigabeelement kann z. B. durch das Bewegen der Gehäuseteile bzw. -abschnitte relativ zu einander von der Sperrposition in die Zwischenposition verschoben werden. In der Zwischenposition kann das Schaltglied in Form eines Freigabeelements mit einer Sicherungsvorrichtung wie etwa einem Einstech-Blockierelement zusammenwirken, welche das Treibelement bzw. die Vortriebsstruktur in einer gesicherten Position festhält. Ist das Freigabeelement in der Zwischenposition kann es durch das Auslöseelement weiter in die Freigabeposition bewegt werden, in der das Treibelement frei gegeben wird. Hierfür kann das Auslöseelement eine Führungseinrichtung aufweisen, die mit dem Freigabeelement derart zusammenwirkt, dass es in die Freigabeposition bewegt wird. Die Führungseinrichtung kann z. B. durch eine relativ zur Längsachse des Autoinjektors schräge Fläche am Freigabeelement, vorzugsweise aber am Auslöseelement vorgesehen sein. Beim Betätigen des Auslöseelement kommt eine Kante des Freigabeelement an der schrägen Fläche des Auslöseelement zu liegen und wird bei Betätigung des Auslöseelements entlang der Schräge in die Freigabeposition verschoben.

Solange das Freigabeelement in der Sperrposition ist bewirkt eine Betätigung des Auslöseelements keinerlei Auslösung im Autoinjektor. Vorzugsweise ist das Auslöseelement durch eine Feder in einer aus dem Autoinjektor hervorstehenden Position vorgespannt. Die Auslösung des Autoinjektors erfolgt auch bei dieser Ausführungsart in zwei von einander unabhängigen, entkoppelten Betätigungsschritten. Ein versehentliches Auslösen des Autoinjektors ist daher nicht möglich.

Die Sicherungs- bzw. Entsicherungsvorrichtung und das Auslöseelement bewegen sich zu einander in Längsrichtung nicht. Ihre Bewegung verläuft in einer Ebene quer zur Längsachse des Autoinjektors, so dass für diese Bauteile kein zusätzlicher Bewegungsspielraum in Längsrichtung des Autoinjektors vorgesehen werden muss. Der Autoinjektor kann daher insgesamt kürzer gestaltet werden.

Nach einer weiteren Ausführungsart der Erfindung ist das Auslöseelement in Längsrichtung des Autoinjektors bewegbar. Dies erlaubt eine einfache Betätigung der Auslöseeinrichtung an einem Ende des Autoinjektors. Bei dieser Ausführungsart hindert das Freigabeelement in seiner Sperrposition das Auslöseelement daran, sich zu bewegen. Damit wird sichergestellt, dass der Autoinjektor zuerst aufgesetzt und erst dann das Betätigungselement gedrückt wird. Nach einer Ausführungsart ist das Freigabeelement in einer Ebene quer zur Längsrichtung des Autoinjektors bewegbar. Dabei kann das Freigabeelement nach einer Variante so geführt sein, dass es von der Sperrposition in die Freigabeposition eine translatorische Bewegung ausführt. Dabei hat das Freigabeelement bevorzugt eine schlüssellochförmige Öffnung und am Treibelement ist eine Ringnut vorhanden, in die der schmale Teil dieser schlüssellochförmigen Öffnung in der Sperrposition des Freigabeelements eingreift. Nach einer anderen Variante ist das Freigabeelement so geführt, dass es von der Sperrposition in die Freigabeposition eine rotatorische Bewegung ausführt. Dabei hat das Freigabeelement eine nicht kreisrunde Öffnung und am Treibelement ist ein Kopf mit einem nicht kreisrunden Querschnitt vorhanden, welcher nur in der Freigabeposition des Freigabeelements durch die Öffnung passt. Beide Varianten sind mit wenigen Einzelteilen realisierbar und erlauben es, einen kostengünstigen und betriebssicheren Autoinjektor zu bauen.

Bevorzugte Merkmale werden auch in den Untersprüchen und deren Kombinationen beschrieben, wobei die durch die Ansprüche beschriebene Merkmale und die vorstehend geschilderten einander wechselseitig vorteilhaft ergänzen.

Ausführungsbeispiele werden nachfolgend anhand von Figuren erläutert. An den Ausführungsbespielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur1: ein erstes Ausführungsbeispiel eines Autoinjektors in einem Ausgangszustand vor einer Injektion,
- Figur 2: einen Nadelschutz, ein Schaltglied und ein Auslöseglied des Autoinjektors in dem Ausgangszustand,
- Figur 3: den Querschnitt A-A der Figur 1
- Figur 4: den Autoinjektor des ersten Ausführungsbeispiels aufgesetzt auf eine Einstechstelle vor einer Auslösung,
- Figur 5: den Nadelschutz, das Schaltglied und das Auslöseglied des Autoinjektors in dem Zustand der Figur 4
- Figur 6: den Querschnitt B-B der Figur 4
- Figur 7: den Autoinjektor des ersten Ausführungsbeipiels aufgesetzt auf die Einstechstelle im ausgelösten Zustand.
- Figur 8: den Nadelschutz, das Schaltglied und das Auslöseglied im ausgelösten Zustand,
- Figur 9: den Querschnitt C-C der Figur 7,
- Figur 10: ein zweites Ausführungsbeispiel eines Autoinjektors in einem Ausgangszustand,
- Figur 11: einen Längsschnitt durch eine weitere Ausführungsart eines Autoinjektors nach der Erfindung im Anlieferungszustand und
- Figuren 12a bis 12c: drei Positionen des Freigabeelements im Querschnitt für die Ausführungsart aus Figur 11.

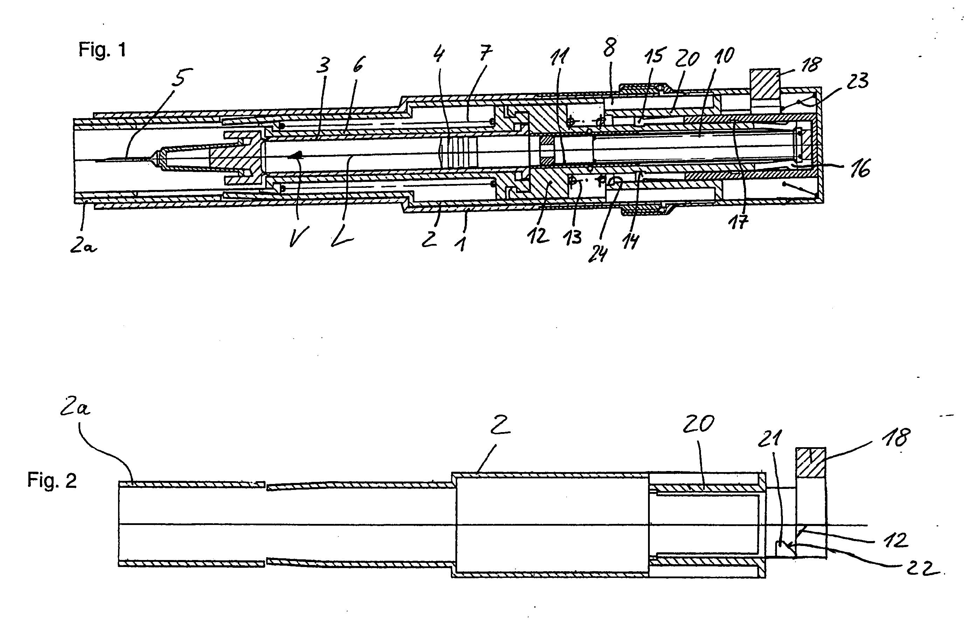

Figur 1 zeigt einen Autoinjektor nach einem ersten Ausführungsbeispiel. Der Autoinjektor weist die Form eines Injektionspen auf. Er umfasst einen hülsenförmigen proximalen Gehäuseabschnitt 1, der als Griffstück dient, und einen hülsenförmigen distalen Gehäuseabschnitt 2, der an seinem distalen Ende einen Nadelschutz 2a bildet. Der Gehäuseabschnitt 2 ist in dem Gehäuseabschnitt 1 entlang einer gemeinsamen Längsachse L axial linear geführt.

Der Gehäuseabschnitt 2 nimmt ein Reservoir 3 auf, das mit einem zu injizierbaren Produkt, beispielsweise Insulin, gefüllt ist. Das Reservoir 3 ist ein Behältnis, im Ausführungsbeispiel eine übliche Ampulle, in dem ein Förderelement 4 in Form eines Kolbens aufgenommen ist. Durch eine Axialbewegung des Förderelements 4 in eine Vortriebsrichtung V wird Produkt durch einen Auslass des Reservoirs 3 und eine mit dem Reservoir 3 verbundene Injektionsnadel 5 ausgeschüttet. Die Injektionsnadel 5 ist an dem distalen Ende des Reservoirs 3 befestigt und ragt mit ihrer freien Nadelspitze in die Vortriebsrichtung V vor. In dem in Figur 1 gezeigten Ausgangszustand des Autoinjektors umgibt der Nadelschutz 2a die Injektionsnadel 5 bis über deren Nadelspitze hinaus. Dies entspricht einer axialen Schutzposition des Nadelschutzes 2a. Das Reservoir 3 ist in einem Reservoirhalter 6 zentriert gehalten und wird von dem Reservoirhalter 6 in die Vortriebsrichtung V abgestützt. Der Gehäuseabschnitt 2 und damit dessen Nadelschutz 2a ist über eine Rückstellfeder 7 elastisch in die Vortriebsrichtung V gespannt. Er kann gegen die Kraft der Feder 7 relativ zu dem Gehäuseabschnitt 2 gegen die Vortriebsrichtung V bewegt werden. Im Ausführungsbeispiel stützt sich die Feder 7 in die Vortriebsrichtung V unmittelbar an dem Gehäuseabschnitt 2 und gegen die Vortriebsrichtung V unmittelbar an dem Reservoirhalter 6 ab.

In dem Gehäuseabschnitt 1 ist eine Antriebseinrichtung aufgenommen und axial bewegbar gelagert. Die Antriebseinrichtung umfasst einen Ausschüttantrieb für die Ausschüttung des Produkts und einen Einstechantrieb für das Vorstechen der Injektionsnadel 5. Eine Ausschüttfeder 10 bildet den Ausschüttantrieb. Die Ausschüttfeder 10 wirkt über eine Ausschüttstruktur 11, die im Ausführungsbeispiel als Ausschütthülse geformt ist, für die Förderung des Produkts auf das Förderelement 4. Aufgrund der Ausbildung des Förderelements 4 als Translationskolben bildet die Ausschüttstruktur 11 eine Kolbenstange. Die Ausschüttfeder 10 spannt die Ausschüttstruktur 11 in die Vortriebsrichtung V. Die Ausschüttstruktur 11 befindet sich in dem Ausgangszustand des Autoinjektors jedoch in einem Halteeingriff, durch den eine Vortriebsbewegung der Ausschüttstruktur 11 verhindert wird.

Auch der Einstechantrieb ist als Feder gebildet, nämlich als Einstechfeder 13. Auch die Einstechfeder 13 ist in dem Ausgangszustand des Autoinjektors in die Vortriebsrichtung V gespannt. Sie beaufschlagt mit ihrer Elastizitätskraft eine Vortriebsstruktur 12, die gegen die Kraft der Enstechfeder 13 in einem Halteeingriff an einer Vortriebsbewegung relativ zu dem Gehäuseabschnitt 1 gehindert ist. Die Vortriebsstruktur 12 umgibt die Ausschüttstruktur 11 und lagert und führt diese axial linear. Sowohl die Ausschüttfeder 10 als auch die Einstechfeder 13 sind gegen die Vortriebsrichtung V an dem Gehäuseabschnitt 1 abgestützt wobei sich die Einstechfeder 13 unmittelbar an einer Lagerstruktur 8 abstützt, die axial und radial nicht beweglich und verdrehfrei und in diesem Sinne fest mit dem Gehäuseabschnitt 1 verbunden ist. Die Lagerstruktur 8 bildet auch eine axiale Linearführung für die Vortriebsstruktur 12.

Den Halteeingriff der Vortriebsstruktur 12 bewirkt ein Einstech-Blockierelement 15, das als elastischer Schnapper gebildet ist und einen Vorsprung aufweist, mit dem es in eine Ausnehmung 14 ragt, die an einer Aussenmantelfläche der hülsenförmigen Vortriebsstruktur 12 gebildet ist. Das Einstech-Blockierelement 15 ist axial relativ zu dem Gehäuseabschnitt 1 nicht beweglich. Um den Umfang eines Hülsenkörpers sind mehrere der Einstech-Blockierelemente 15 je als axiale Federzunge gebildet.

Für den Halteeingriff der Ausschüttstruktur 11 bildet die Vortriebsstruktur 12 an ihrem proximalen Ende ebenfalls mehrere Federzungen, die als Ausschütt-Blockierelemente 16 wirken, indem sie je einen Vortriebsanschlag für die Ausschüttstruktur 11 bilden. Der gleiche Hülsenkörper, der die Einstech-Blockierelemente 15 bildet, drückt die Ausschütt-Blockierelemente 16 nach radial einwärts in den Halteingriff mit der Ausschüttstruktur 11.

Die Einstech-Blockerelemente 15 werden von einem Schaltglied 20 nach radial einwärts in den Halteeingriff, d.h. in die Ausnehmungen 14 oder die als umlaufende Nut gebildete Ausnehmung 14 der Vortriebsstruktur 12 gedrückt. Das Schaltglied 20 weist hierfür einen Hülsenabschnitt auf, der die Vortriebsstruktur 12 und die Einstech-Blockierelemente 15 umgibt und sie in die Ausnehmungen 14 oder die umlaufenden Ausnehmung 14 drückt. Die Lagerstruktur 8 lagert das Schaltglied 20 axial beweglich. Die Lagerstruktur 8 und/oder der die Einstech-Blockierelemente 15 bildende Hülsenkörper führen das Schaltglied 20 axial linear. Das Schaltglied 20 ist in einem axialen Druckkontakt mit dem Gehäuseabschnitt 2, so dass eine Bewegung des Gehäuseabschnitts 2 gegen die Vortriebsrichtung V eine ebensolche Bewegung des Schaltglieds 20 bewirkt. Schliesslich ist das Schaltglied 20 über eine Rückstellfeder 23 gegen die Vortriebsrichtung V an dem Gehäuseabschnitt 1 abgestützt. In dem Ausgangszustand nimmt das Schaltglied 20 eine axiale Verriegelungsposition ein, in der es die Einstech-Blockierelemente 15 in ihrem Halteeingriff mit der Vortriebsstruktur 12 sperrt.

In einem proximalen Abschnitt des Gehäuseabschnitts 1 ragt aus dessen Mantelfläche ein Auslöseglied 18 nach radial auswärts vor. Das Auslöseglied 18 ist in den Gehäuseabschnitt 1 tiefer hineindrückbar, vorzugsweise durch radialen Druck betätigbar, und bewegt sich durch die Betätigung in eine Richtung quer, im Ausführungsbeispiel radial, zu einer in die Vortriebsrichtung V weisenden Mittellängsachse L des Autoinjektors.

Die Figur 2 zeigt den Gehäuseabschnitt 2 mit dem Nadelschutz 2a, das Schaltglied 20 und Auslöseglied 18 herausgelöst aus dem Autoinjektor der Figur 1 in den Positionen relativ zueinander, die sie in dem Ausgangszustand des Autoinjektors einnehmen. Zwischen dem Gehäuseabschnitt 2 und dem Schaltglied 20 besteht lediglich der axiale Druckkontakt. Das Auslöseglied 18 und das Schaltglied 20 sind nicht in Eingriff. Zu erkennen ist jedoch insbesondere eine Schaltkurve 19, die zu der Vortriebsrichtung V und zu der Richtung der Querbewegung des Auslöseglieds 18 geneigt ist. Die Vortriebsrichtung V und die Richtung der Querbewegung, d.h. die Richtung der Auslösebewegung des Auslöseglieds 18 weisen senkrecht zueinander. Die Schaltkurve 19 weist gegen die Vortriebsrichtung V. Das Schaltglied 20 ist mit einem Nocken 21 versehen, der in einem Koppeleingriff des Schaltglieds 20 mit dem Auslöseglied 18 ein Eingriffsglied bildet, das an der Schaltkurve 19 abgleitet, wenn das Auslöseglied 18 seine Auslösebewegung ausführt. Das Schaltglied 20 ist ferner ebenfalls mit einer Schaltkurve 22 versehen, im Ausführungsbeispiel an einer rückwärtigen Fläche des Nockens 21. Die Schaltkurve 22 hat für die Auslösung des Autoinjektors keine Funktion. Sie dient lediglich der Korrektur der Stellung des Auslöseglieds 18, falls dieses im Ausgangszustand des Autoinjektors versehentlich gedrückt wurde. Beide Schaltkurven 19 und 22 verlaufen gerade, d.h. einfach nur schräg. Ein nichtlinearer Verlauf ist jedoch grundsätzlich ebenfalls realisierbar.

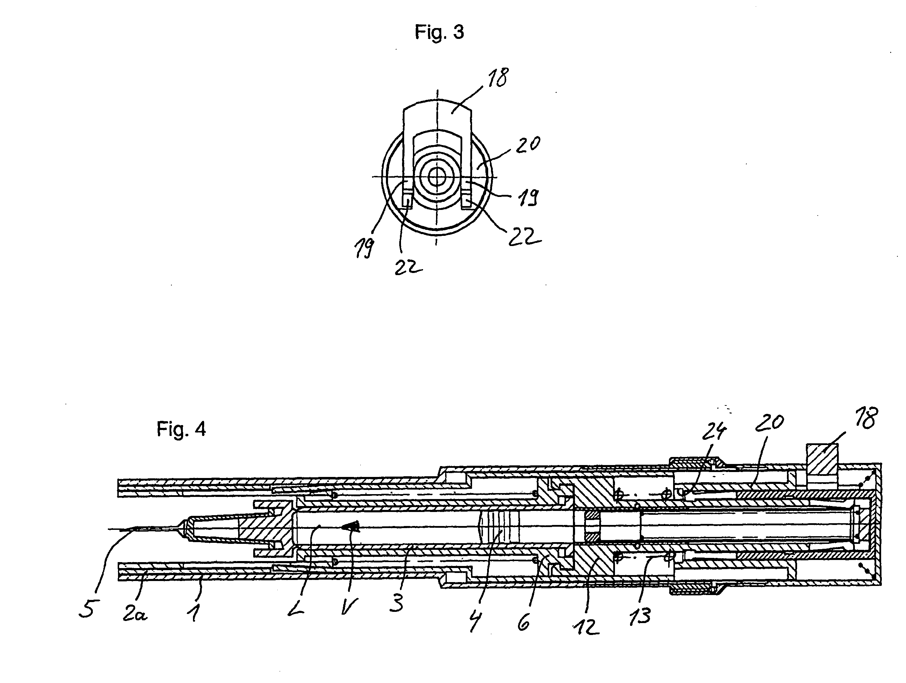

Figur 3 zeigt in dem Querschnitt A-A der Figur 1 insbesondere das Auslöseglied 18 und das Schaltglied 20 sowie deren Schaltkurven 19 und 22.

Wird der Autoinjektor an einer gewünschten Einstechstelle auf der Haut aufgesetzt, so wird in einem ersten Schritt der Injektion durch Druck gegen die Einstechstelle der Nadelschutz 2a und damit der gesamte Gehäuseabschnitt 2 relativ zu dem Gehäuseabschnitt 1 nach proximal bewegt, d.h. tiefer in den Gehäuseabschnitt 1 hineinbewegt, bis das distale Ende des Nadelschutzes 2a und das distale Ende des Gehäuseabschnitts 1 sich auf der gleicher axialen Höhe befinden.

Figur 4 zeigt den Autoinjektor am Ende dieser ersten Phase der Injektion. Durch die Rückwärtsbewegung des Nadelschutzes 2a in die in Figur 4 gezeigte Rückzugsposition, in der die Nadelspitze unmittelbar über der Haut steht, hat der Nadelschutz 2a bzw. der Gehäuseabschnitt 2 das Schaltglied 20 um den Weg der eigenen Rückwärtsbewegung relativ zu dem Gehäuseabschnitt 1 und insbesondere relativ zu dem Auslöseglied 18 zurückbewegt.

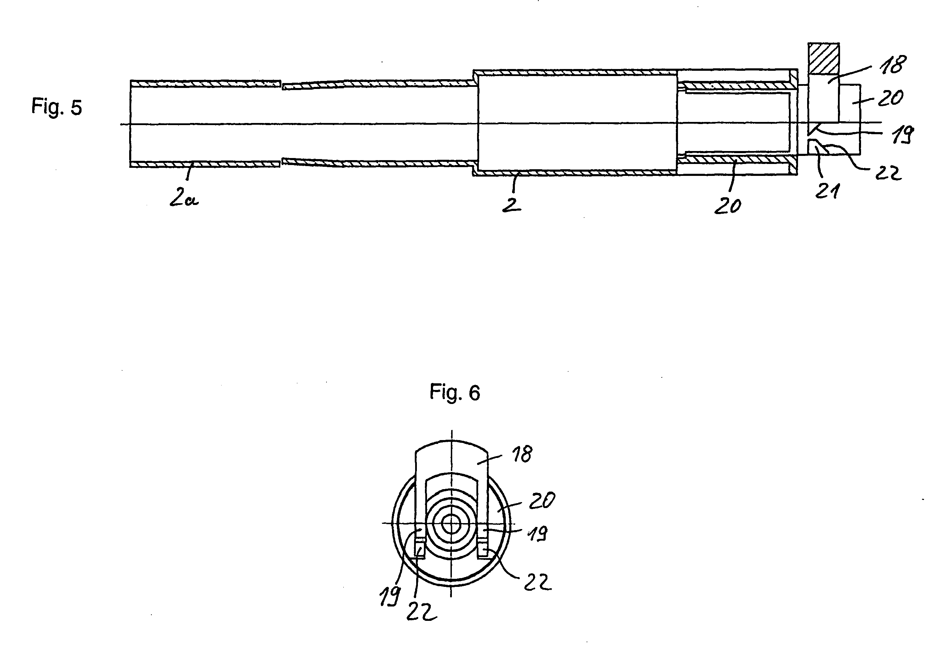

Figur 5 zeigt die jetzt eingenommenen Relativpositionen des Gehäuseabschnitts 2, des Schaltglieds 20 und des Auslöseglieds 18 besonders deutlich. Die Axialposition des Schaltglieds 20 ist relativ zu dem Auslöseglied 18 so, dass die Schaltkurve 19 im Verlauf einer Auslösebewegung des Auslöseglieds 18, d.h. bei einer Druckbetätigung des Auslöseglieds 18, in einen Druckgleitkontakt mit dem Nocken 21 gelangt und im Druckgleitkontakt an dem Nocken 21 entlang gleitet. Die jetzt erreichte Axialposition des Schaltglieds 20 wird daher als Koppelposition bezeichnet.

Wird bei in der Koppelposition befindlichem Schaltglied 20 die Injektion durch Druck auf das Auslöseglied 18 ausgelöst, so bewegt sich dieses relativ zu dem Gehäuseabschnitt 1 und insbesondere relativ zu dem Schaltglied 20 auf dessen Nocken 21 zu, gelangt mit dem Nocken 21 in den besagten Gleitdruckkontakt und drückt aufgrund des geneigten Verlaufs der Schaltkurve 19 den Nocken 21 und damit zusammen das Schaltglied 20 gegen die Vortriebsrichtung V, d.h. es zieht das Schaltglied 20 nach proximal. Der Nocken 21 bildet für diese Axialbewegung des Schaltglieds 20 ein Eingriffsglied, das an der Schaltkurve 19 bei dem Eindrücken des Auslöseglieds 18 entlang gleitet. Da das Auslöseglied 18 geführt ist und relativ zu dem Gehäuseabschnitt 1 keine Axialbewegung, sondern nur die radiale Auslösebewegung ausführt, wird das Schaltglied 20 über seinen Nocken 21 gegen die Vortriebsrichtung V weiter zurückgezogen bis in eine axiale Endposition.

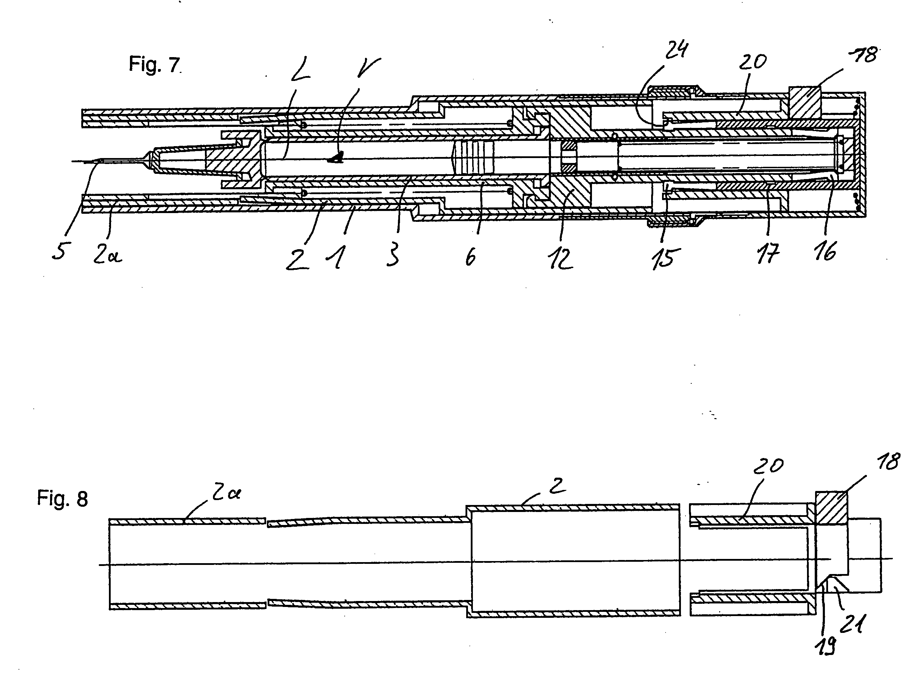

Figur 7 zeigt den Autoinjektor nach der Auslösung, d.h. das Auslöseglied 18 hat seine Auslösebewegung ausgeführt und das Schaltglied 20 nimmt seine proximale Endposition relativ zu dem Auslöseglied 18 und insbesondere zu den Einstech-Blockierelementen 15 ein. Das Schaltglied 20 ist in seinem Hülsenabschnitt, mit dem es bislang die Einstech-Blockierelemente 15 in den Halteeingriff mit der Vortriebstruktur 12 gedrückt hat, eine Ausnehmung 24 auf, im Ausführungsbeispiel in Form einer an der Innenmantelfläche des Hülsenabschnitts des Schaltglieds 20 umlaufenden Verbreiterung, mit der das Schaltglied distal endet. In der proximalen Endposition des Schaltglieds 20 schnappen die Einstech-Blockierelemente 15 aufgrund ihrer eigenen elastischen Rückstellkräfte aus dem Halteingriff mit der Vortriebstruktur 12 nach radial aussen in die Ausnehmung 24 vor. Der Halteeingriff der Vortreibstruktur 12 ist damit gelöst, und die Vortreibstruktur 12 wird von der gespannten Einsteckfeder 13 in die Vortriebsrichtung V vorgetrieben. Sie drückt bei ihrem eigenen Vortrieb gegen den Reservoirhalter 6, der zusammen mit dem darin aufgenommen Reservoir 3 in die Vortriebsrichtung V bewegt wird. Bei der Vortriebsbewegung sticht die Injektionsnadel 5 aus dem Gehäuseabschnitt 1 und 2 vor und in und vorzugsweise durch die Haut. Die Vortriebsbewegung der Vortriebsstruktur 12 ist durch einen Anschlag begrenzt.

Sobald die Vortriebsbewegung der Vortriebsstruktur 12 und damit die Vorstechbewegung der Injektionsnadel 5 beendet ist, gelangen die Ausschütt-Blockierelemente 16 in eine Ausnehmung 17 des Hülsenkörpers, der auch die Einstech-Blockierelemente 15 bildet, und schnappen in die Ausnehmung 17 vor. Durch das Vorschnappen der Ausschütt-Blockierelemente 16 löst sich der Halteeingriff der Ausschüttstruktur 11 und diese schiebt relativ zu der Vortriebstruktur 12 nun in die Vortriebsrichtung V vor. Die Ausschüttstruktur 11 gelangt dabei in Kontakt mit dem Förderelement 4 und schiebt dieses in dem Reservoir 3 auf dessen Auslass vor. Hierdurch wird das Produkt aus dem Reservoir 3 und durch die Injektionsnadel 5 ausgeschüttet und verabreicht.

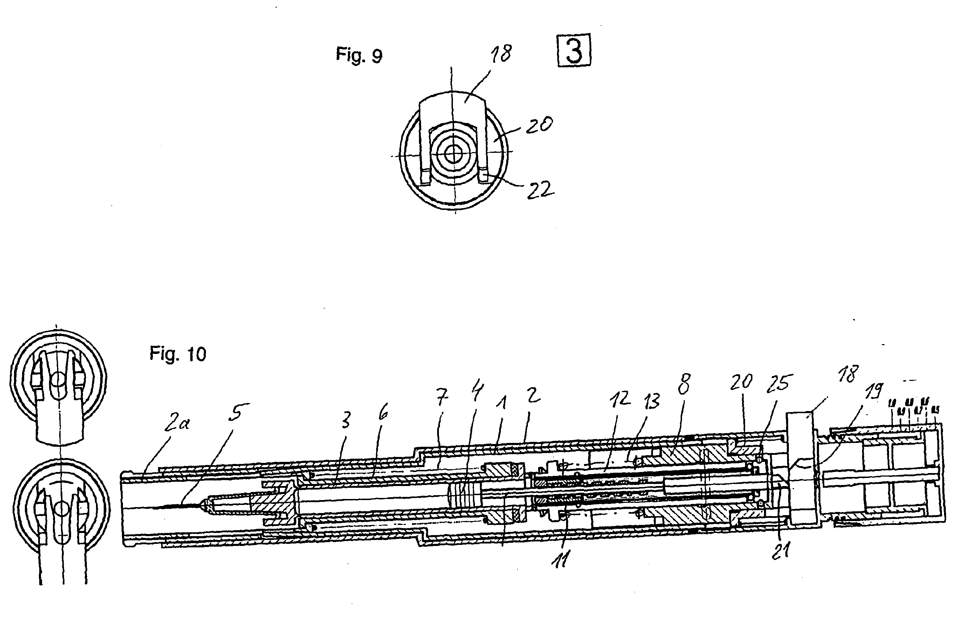

Figur 8 zeigt nochmals einzeln den Gehäuseabschnitt 2, das Schaltglied 20 und das Auslöseglied 18 in denjenigen Axialpositionen, die sie nach der Auslösung relativ zueinander einnehmen. Figur 9 zeigt den gleichen Zustand in dem Querschnitt C-C der Figur 7.

Figur 10 zeigt eine Autoinjektor eines zweiten Ausführungsbeispiels. Der Autoinjektor des zweiten Ausführungsbeispiels unterscheidet sich von demjenigen des ersten Ausführungsbeispiels lediglich in Bezug auf seine Einstech-Blockierelemente, die im zweiten Ausführungsbeispiel mit 25 bezeichnet sind. Die Blockierelemente 25 sind Kugeln oder Zylinderstifte, die ebenfalls nach radial auswärts gespannt sind und von dem Schaltglied 20 gegen eine Bewegung aus dem Halteeingriff gesperrt werden. Insoweit entspricht insbesondere das Schaltglied 20 des zweiten Ausführungsbeispiels dem des ersten Ausführungsbeispiels. Auch bezüglich des Zusammenwirkens mit dem Nadelschutz 2a und dem Auslöseglied 18 bestehen keine Unterschiede. Unterschiede bestehen lediglich in anderer Hinsicht und haben mit der Auslösemechanik, wie sie der Nadelschutz 2a bzw. der Gehäuseabschnitt 2, das Schaltglied 20 und das Auslöseglied 18 bilden, nichts zu tun. Diesbezüglich gelten vielmehr die Ausführungen zum ersten Ausführungsbeispiel gleichermaßen.

Die Ausführungsart der Figuren 1 bis 10 kann auch wie folgt beschrieben werden. Bei dem Autoinjektor ist das Auslöseelement 18 in einer Ebene quer zur Längsrichtung des Autoinjektors und das Freigabeelement 20 in Längsrichtung des Autoinjektors bewegbar ist. Das Freigabeelement 20 wird von einer Hülse gebildet, die gegenüber dem vorderen, distalen und dem hinteren proximalen Gehäuseteil 1 und 2 axial verschiebbar ist und innerhalb dieser Gehäuseteile angeordnet ist. Das Auslöseelement 18 wird durch einen seitlichen vom Autoinjektor abragenden Knopf gebildet, der im wesentlichen senkrecht zur Längsachse des Autoinjektors angeordnet ist. In den Figuren 1 und 2 ist das Freigabeelement 20 in der Sperrposition bzw. Schutzposition, d. h. in einer entfernt vom Auslöseelement 18 gelegenen Position, und wird durch die Konusfeder 23 in dieser Sperrposition vorgespannt. Dabei wirkt das Freigabeelement 20 mit einer Sicherungsvorrichtung in Form der zwei sich gegenüberliegenden vorgespannten Sicherungsarmen 15 zusammen, die in eine Vertiefung 14 am Treibelement 12 eingreifen und dieses dadurch gegen Vorschub sichert. Natürlich könnte auch nur ein Sicherungsarm oder mehr als zwei Sicherungsarme verwendet werden. Die Sicherungsarme 15 werden durch die Innenfläche der Hülse des Freigabeelements 20 in die Vertiefungen 14des Treibelements 12 vorgespannt. Die Sicherungsvorrichtung könnte z. B. auch durch Kugeln oder ein vorgespanntes Ringelement gegeben sein, welche mit dem Treibelement in einer Sperrbeziehung stehen.

In den Figuren 4 und 5 ist das Freigabeelement 20 in einer Zwischenposition bzw. Koppelposition gezeigt. Das vordere Gehäuseteil 2 wird relativ zum hinteren Gehäuseteil 1 in den Autoinjektor hinein verschoben, z. B. beim Aufsetzten auf eine Injektionsstelle auf der Haut eines Patienten. Das Freigabeelement 20 grenzt an das vordere Gehäuseteil 2 an und wird gemeinsam mit diesem relativ zum hintern Gehäuseteil 3 in die Zwischenposition verschoben. Die Sicherungsarme 15 gleiten entlang der Innenfläche der Hülse des Freigabeelements 20 und werden von der Hülse des Freigabeelements 20 immer noch in die Vertiefungen am Treibelement 12 gedrückt, so dass sie dieses gegen Vorschub sichern. Wird der Autoinjektor wieder von der Injektionsstelle abgenommen, wird das Freigabeelement 20 durch die Konusfeder 23 wieder in die Sperrposition zurück geschoben.

Das Freigabeelement 20 ist in der Zwischenposition in den Bewegungsbereich des Auslöseelements 18 gelangt und kann nun mit einer Führungseinrichtung am Auslöseelement 18 zusammenwirken und durch dieses in eine Freigabeposition bewegt werden. Die Führungseinrichtung ist in dem gezeigten Ausführungsbeispiel durch die schräg zur Längsachse des Autoinjektors verlaufende Fläche 22 am Auslöseelement 18 vorgesehen, die eine Schaltkurve bildet. Alternativ könnte eine analoge Fläche, bzw. eine Führungseinrichtung, auch am Freigabeelement 20 angeordnet werden. An der Hülse des Freigabeelements 20 ist eine Verlängerung in Richtung des Auslöseelements 18 vorgesehen, die eine Nase, bzw. einen Nocken 21 aufweist, welche in der Zwischenposition der schrägen Fläche 22 des Auslöseelements 18 in radialer Richtung der Längsachse des Autoinjektors zu liegen kommt, wie am besten aus Figur 5 ersichtlich ist.

Die Figuren 7 und 8 zeigen das Freigabeelement 20 in einer Freigabeposition. Ist das Freigabeelement 20 in der Zwischenposition, kann das Auslöseelement 18 mit dem Freigabeelement 20 in Wechselwirkung treten. Bei Betätigung des Auslöseelements 18 wird die schräge Fläche 22 auf die Nase 21 des Freigabeelements 20 zu bewegt. Die Nase 21 kommt auf der schrägen Fläche 22 zu liegen und gleitet bei weiterem Eindrücken des Auslöseelements 18 entlang der schrägen Fläche, so dass das Freigabeelement 20 aufgrund der schrägen Geometrie in Richtung des Auslöseelements 18 gezogen, bzw. verschoben, wird bis das Freigabeelement 20 in der Freigabeposition ist. In der Freigabeposition kommt ein Bereich mit einem erweiterten Querschnitt der Hülse des Freigabeelements 20 gegenüber den Sicherungsarmen 15 zu liegen, so dass sich diese aufgrund ihrer Vorspannung radial nach aussen aus den Vertiefungen 14 des Treibelements 12 bewegen und das Treibelement 12 frei geben. Das Treibelement kann nun auf den Wirkstoffbehälter 3, bzw. die Injektionsnadel 5, wirken und diese über das vordere Ende der Gehäuseteile 1 und 2 hinaus schieben und in einer Injektionsstelle einstechen.

Bei dieser Ausführungsart kann eine Auslösung nur erfolgen, wenn die vordere Hülse 2 in die hintere Hülse 1 hinein verschoben ist und gleichzeitig der Auslöseknopf 18 gedrückt wird.

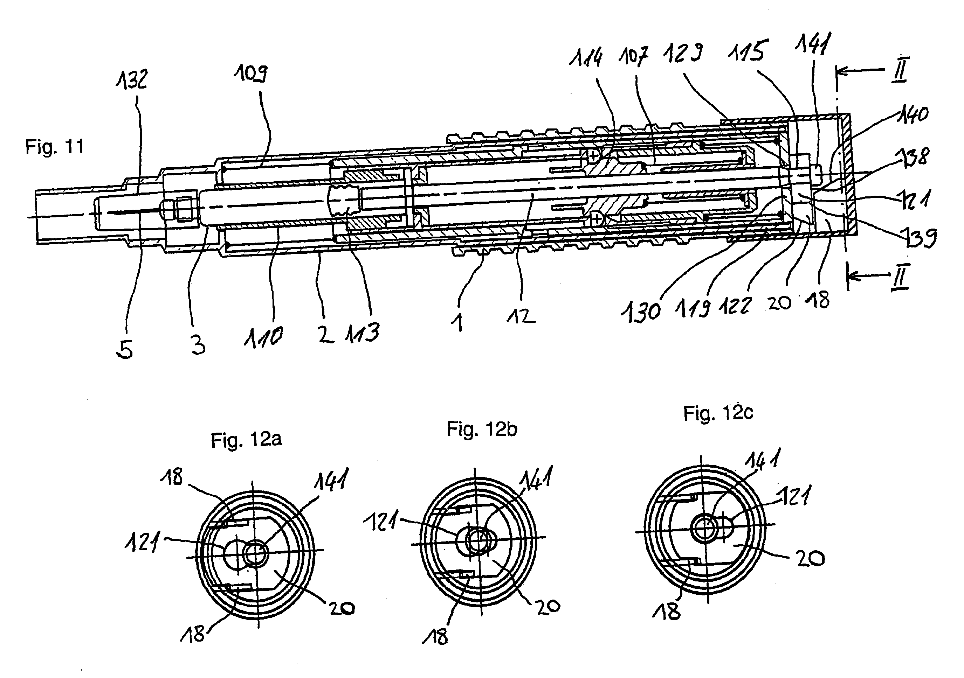

Die Figuren 11 und 12a bis 12c zeigen eine weitere Ausführungsform eines Autoinjektors nach der vorliegenden Erfindung.

Der in Figur 11 in einem Längsschnitt dargestellte Autoinjektor ist zum einmaligen Gebrauch bestimmt und weist ein Gehäuse auf, das aus einem hinteren Gehäuseteil 1 und einem vorderen Gehäuseteil 2 besteht, die aus der in Figur 11 dargestellten Lage entgegen der Kraft einer Feder 109 gegeneinander bewegbar sind. Im Inneren des Autoinjektors ist ein mit dem zu injizierenden Wirkstoff gefüllter Behälter 3 in einer Aufnahmehülse 110 aufgenommen. Der Behälter 3 trägt an seinem vorderen Ende eine Injektionsnadel 5 und ist zwecks Einstechens der Injektionsnadel 5 in die Haut eines Patienten mitsamt der Aufnahmehülse 110 durch die Kraft einer Triebfeder 107 im Gehäuse axial verschiebbar. Die Injektionsnadel 5 ist dabei nicht erfindungswesentlich, der hier beschriebene Auslösemechanismus kann prinzipiell auch bei einem nadellosen Autoinjektor verwendet werden. Eine Vortriebsstruktur, die ein stangenförmiges Treibelement 12 umfasst, greift mit seinem vorderen, der Injektionsnadel 5 zugewandten Ende in den Behälter 3 und ist dort mit einem Kolben 4 verbunden, welcher zum Ausschütten des im Behälter 3 enthaltenen Wirkstoffs dient. Zwischen den beiden Enden des Treibelements 12 ist dieses mit einem Übertragungsteil 114 verbunden, durch welchen die Kraft der Triebfeder 107 auf das Treibelement 12 übertragen wird. An seinem hinteren, der Injektionsnadel 5 abgewandten Ende hat das Treibelement 12 eine Ringnut 115, deren Funktion weiter unten erläutert wird.

Der Autoinjektor ist in Figur 11 in gespanntem und gebrauchsbereitem Zustand dargestellt. Das durch die Kraft der komprimierten Triebfeder 107 in Richtung zum Behälter 3 hin vorgespannte Treibelement 12 wird an seinem hinteren Ende durch ein Freigabeelement 20 festgehalten, indem dieses in die Ringnut 115 eingreift. Das Freigabeelement 20 ruht auf einer Halteplatte 130 und ist quer zur Längsachse des Autoinjektors beweglich, vorzugsweise entgegen der Kraft einer nicht dargestellten Feder. Das Treibelement 12 ragt dabei mit seinem hinteren Ende durch eine Öffnung 129 in dieser Halteplatte 130. Ein Auslöseelement 18 ist in einer Art Deckel vorhanden, der über das hintere Ende des Autoinjektors gestülpt ist und in dessen axialer Richtung, vorzugsweise entgegen Kraft einer nicht dargestellten Feder betätigbar ist. Wie der Figur 11 deutlich zu entnehmen ist, kann das Auslöseelement 18 in der dargestellten Betriebslage des Autoinjektors nicht betätigt werden, weil es an der ebenen Fläche 138 des Freigabeelements 20 ansteht.

Zur Benutzung des Autoinjektors ist zuerst die Nadelschutzkappe 132 zu entfernen, welche die Injektionsnadel 5 steril hält. Dann wird der Autoinjektor am hinteren Gehäuseteil 1 ergriffen, mit dem vorderen Gehäuseteil 2 auf die Haut des Patienten aufgesetzt und leicht angedrückt, wobei sich der vordere Gehäuseteil 2 gegen die Kraft der Feder 109 relativ zum hinteren Gehäuseteil 1 verschiebt. Dabei kommt eine am vorderen Gehäuseteil 2 angeformte Zunge 119 an einer schrägen Fläche 122 des Freigabeelements 20 zur Anlage und bewegt das Freigabeelement quer zur Längsrichtung des Autoinjektors in eine Zwischenposition. Der dabei zurückgelegte Weg des Freigabeelements genügt nicht, um das Treibelement 12 freizugeben. Jedoch kommt eine Kante 139 am Freigabeelement 20 in den Bereich einer am Auslöseelement 18 vorhandenen Schrägfläche 140, so dass nun das Auslöseelement 18 in axialer Richtung des Autoinjektors betätigt werden kann. Durch den Kontakt der Schrägfläche 140 mit der Kante 139 wird das Freigabeelement weiter bewegt, bis es seine Freigabeposition erreicht, in der es das Treibelement 12 freigibt, so dass eine Injektion ausgelöst wird.

Die Figuren 12a bis 12c zeigen in einem Querschnitt entlang der Linie II - II in Figur 11 die Funktion des Freigabeelements 20. Dabei zeigt die Figur 12a das Freigabeelement 20 in der gleichen Position wie Figur 11, nämlich in seiner Sperrposition. Im Freigabeelement 20 ist eine Öffnung 121 vorhanden, welche die Form eines Schlüssellochs hat. In der Sperrposition gemäss Figur 12a greift der schmalere Teil der Öffnung 121 in die Ringnut 115 des Treibelements 12 und hält dieses dadurch fest. Figur 12b zeigt die Zwischenposition, in der die Zunge 119 das Freigabeelement 20 in seine Zwischenposition verschoben hat. Ein axial an die Ringnut 115 anschliessender Kopf 141 des Treibelements befindet sich nun am Übergang zwischen dem schmalen Teil und dem breiten Teil der Öffnung 121 im Freigabeelement, wodurch das Treibelement 12 immer noch in seiner durch die Feder 107 belasteten Position festgehalten wird. Schliesslich zeigt Figur 12c die Freigabeposition des Freigabeelements 20. Der breitere Teil der Öffnung 121 hat sich in den Bereich der Ringnut 115 verschoben, so dass nun der Kopf 141 durch den breiteren Teil der Öffnung 121 treten kann und die Injektion ausgelöst wird.

Die oben beschriebene und in den Figuren dargestellte Ausführungsart der Erfindung basiert auf einer translatorischen Bewegung des Freigabeelements 20. In einer anderen, nicht zeichnerisch dargestellten Ausführungsart ist das Freigabeelement 20 rotatorisch bewegbar. Bei einer solchen Ausführungsart ist die Zunge 119 an ihrem zum Freigabeelement 20 gerichteten Ende derart angeschrägt, dass sie beim gegeneinander Bewegen der Gehäuseteile 1, 2 das Freigabeelement um die Längsachse des Autoinjektors dreht. Die Schrägfläche 140 des Auslöseelements 18 ist dabei nicht wie in Figur 11 radial, sondern in Umfangsrichtung ausgerichtet. Das Freigabeelement hat auch gemäss dieser Ausführungsart drei Positionen und wird durch die Zunge 119 von der Sperrposition in die Zwischenposition und durch das Auslöseelement 18 von der Zwischenposition in die Freigabeposition bewegt. Die Öffnung im Freigabeelement und der Kopf 141 des Treibelements 12 haben bei dieser Ausführungsart eine nicht kreisrunde Form. In der Sperrposition und in der Zwischenposition passt der Kopf nicht durch die Öffnung, wogegen er in der Freigabeposition durch die Öffnung passt.

Diese Aufgabe wird demnach gemäss der Erfindung dadurch gelöst, dass das Freigabeelement durch das gegeneinander Verschieben der Gehäuseteile von seiner Sperrposition in eine Zwischenposition bewegbar ist, in der es das Treibelement festhält und dass das Auslöseelement fähig ist, das Freigabeelement aus der Zwischenposition in die Freigabeposition zu bewegen.

Indem weder die Bewegung der Gehäuseteile noch die Betätigung des Auslöseelements für sich allein zu einer Auslösung einer Injektion führen, wird eine besonders gute Sicherheit gegen eine unbeabsichtigte Auslösung erreicht. Zudem wird durch diese Lösung sichergestellt, dass der Autoinjektor auf der Haut eines Patienten aufgesetzt sein muss, bevor das Auslöseelement betätigt werden kann.

### Bezugszeichen:

- 1: proximaler Gehäuseabschnitt, hintere Hülse
- 2: distaler Gehäuseabschnitt, vordere Hülse
- 2a: Nadelschutz
- 3: Reservoir, Wirkstoffbehälter, Behälter
- 4: Förderelement, Kolben
- 5: Injektionsnadel
- 6: Reservoirhalter
- 7: Rückstellfeder
- 8: Lagerstruktur
- 9: -
- 10: Ausschüttantrieb, Ausschüttfeder
- 11: Ausschüttstruktur, Kolbenstange
- 12: Vortriebsstruktur, Treibelement
- 13: Einstechantrieb, Einstechfeder
- 14: Ausnehmung, Vertiefung
- 15: Einstech-Blockierelement, Schnapper, Sicherungsarm
- 16: Ausschütt-Blockierelement
- 17: Ausnehmung, Vertiefung
- 18: Auslöseglied, Auslöseelement, Auslöseknopf
- 19: Schaltkurve
- 20: Schaltglied, Freigabeelement
- 21: Nocken, Nase
- 22: Schaltkurve, schräge Fläche
- 23: Rückstellfeder, Konusfeder
- 24: Ausnehmung
- 25: Einstech-Blockierelement
- L: Längsachse
- V: Vertriebsrichtung
- 107: Triebfeder
- 108: Halteelement
- 109: Feder
- 110: Aufnahmehülse
- 114: Übertragungsteil
- 115: Ringnut
- 116: Ringnut
- 117: Feder
- 118: Feder
- 119: Zunge an 122
- 120: Öffnung in 18
- 121: Öffnung in 20
- 122: schräge Fläche an 20
- 123: Feder
- 124: Innenhülse
- 125: Ausnehmung
- 126: Vertiefung
- 127: Arme von 18
- 128: Halteschenkel
- 129: Öffnung
- 130: Halteplatte
- 131: Haltenase
- 132: Nadelschutzkappe
- 133: Sicherungsdeckel
- 134: Sicherungsstift
- 135: Körper
- 136: Steg
- 137: hülsenförmiges Ende von 122
- 138: ebene Fläche an 20
- 139: Kante an 20
- 140: Schrägfläche an 18
- 141: Kopf an 12
- 142: Rohr
- 143: Schulter
- 144: Verschiebesicherung

## Patentansprüche

1. Autoinjektor mit einem länglichen Gehäuse, in dem ein Behälter (3) für einen Wirkstoff angeordnet ist, wobei das Gehäuse aus mindestens zwei Gehäuseteilen (1, 2) besteht, die relativ zu einander beweglich sind, mit einem Treibelement (12), das durch die Kraft mindestens einer Feder (7) in Injektionsrichtung vorspannbar ist, mit einem Freigabeelement (20), welches von einer Sperrposition, in der das Treibelement (12) in einer vorgespannten Lage gesichert ist in eine Freigabeposition bewegbar ist, in der das Treibelement (12) entsichert ist, und mit einem Auslöseelement (18),
**dadurch gekennzeichnet, dass** das Freigabeelement (20) durch eine Relativbewegung der Gehäuseteile (1, 2) von seiner Sperrposition in eine Zwischenposition bewegbar ist, in der das Treibelement (12) gesichert ist und dass das Auslöseelement (18) fähig ist, das Freigabeelement (20) aus der Zwischenposition in die Freigabeposition zu bewegen.

2. Autoinjektor nach Anspruch 1, **dadurch gekennzeichnet, dass** das Auslöseelement (18) in einer Ebene quer zur Längsrichtung des Autoinjektors und das Freigabeelement (20) in Längsrichtung des Autoinjektors bewegbar ist.

3. Autoinjektor nach einem der vorangehenden Ansprüche, **dadurch gekennzeichnet, dass** das Auslöseelement (18) eine Führungseinrichtung (19, 21, 22) zur Führung des Freigabeelements (20) bei der Bewegung von der Zwischenposition in die Freigabeposition aufweist.

4. Autoinjektor nach einem der vorangehenden Ansprüche, **dadurch gekennzeichnet, dass** das Freigabeelement (20) mit einer Sicherungsvorrichtung (15) zur Sicherung des Treibelements (12) in einer vorgespannten Lage zusammenwirkt.

5. Autoinjektor nach einem der vorangehenden Ansprüche, **dadurch gekennzeichnet, dass** die Sicherungsvorrichtung (15) zur Entsicherung in einer Ebene quer zur Längsrichtung des Autoinjektors beweglich ist.

6. Autoinjektor nach Anspruch 1, **dadurch gekennzeichnet, dass** das Auslöseelement (18) in Längsrichtung des Autoinjektors bewegbar ist und das Freigabelement (20) in einer Ebene quer zu Längsrichtung des Autoinjektors bewegbar ist.

7. Autoinjektor nach einem der vorherigen Ansprüche, **dadurch gekennzeichnet, dass** das Freigabeelement (20) in seiner Sperrposition das Auslöseelement (18) daran hindert, sich zu bewegen.

8. Autoinjektor nach einem der vorherigen Ansprüche, **dadurch gekennzeichnet, dass** das Freigabeelement (20) eine schlüssellochförmige Öffnung (121) hat und dass am Treibelement eine Ringnut (115) vorhanden ist, in die der schmale Teil dieser schlüssellochförmigen Öffnung (121) in der Sperrposition des Freigabeelements (20) eingreift.

9. Autoinjektor nach Anspruch 1, **dadurch gekennzeichnet, dass** das Freigabeelement (20) so geführt ist, dass es von der Sperrposition in die Freigabeposition eine rotatorische Bewegung ausführt.

10. Autoinjektor nach Anspruch 9, **dadurch gekennzeichnet, dass** das Freigabeelement (20) eine nicht kreisrunde Öffnung (121) hat und dass am Treibelement ein Kopf mit einem nicht kreisrunden Querschnitt vorhanden ist, welcher nur in der Freigabeposition des Freigabeelements durch die Öffnung passt.

11. Auslösbares Injektionsgerät, umfassend:
a) einen Gehäuseabschnitt (1)
b) ein Reservoir (3) für ein injizierbares Produkt
c) eine Injektionsnadel (5) mit einer Nadelspitze an einem distalen Ende
d) einen Nadelschutz (2a) der relativ zu dem Gehäuseabschnitt (1) und der Injektionsnadel (5) aus einer Schutzposition, in der er die Injektionsnadel (5) bis über die Nadelspitze umgibt, nach proximal bis in eine Rückzugsposition bewegbar ist,
e) eine Antriebseinrichtung (4,10-13) für eine Injektion, die in einem lösbaren Halteeingriff gegen eine Antriebsbewegung gesperrt ist,
f) ein Schaltglied (20), das mit dem Nadelschutz (2a) so gekoppelt ist, dass die Bewegung des Nadelschutzes (2a) nach proximal eine Bewegung des Schaltglieds (20) aus einer Verriegelungsposition in eine Koppelposition bewirkt,
g) und ein betätigbares Auslöseglied (18) für eine Auslösung der Antriebseinrichtung (4,10-13), das bei seiner Betätigung mit dem Schaltglied (20) gekoppelt wird, wenn das Schaltglied (20) die Koppelposition einnimmt, und eine Bewegung des Schaltglieds (20) aus der Koppelposition in eine Freigabeposition bewirkt,
h) wobei sich der Halteeingriff der Antriebseinrichtung (4,10-13) erst in der Freigabeposition des Schaltglieds (20) löst.

12. Injektionsgerät nach dem vorhergehenden Anspruch, **dadurch gekennzeichnet, dass** die Bewegung des Schaltglieds (20) aus der Verriegelungsposition bis in die Freigabeposition in die gleiche Richtung weist.

13. Injektionsgerät nach einem der vorhergehenden Ansprüche, **dadurch gekennzeichnet, dass** das Schaltglied (20) aus der Verriegelungsposition bis in die Freigabeposition in die proximale Richtung bewegt wird.

14. Injektionsgerät nach einem der vorhergehenden Ansprüche, **dadurch gekennzeichnet, dass** das Schaltglied (20) aus der Verriegelungsposition bis in die Freigabeposition axial linear geführt ist.

15. Injektionsgerät nach einem der vorhergehenden Ansprüche, **dadurch gekennzeichnet, dass** das Auslöseglied (18) zu einer Richtung, in die das Schaltglied (20) in die Freigabeposition bewegt wird, quer bewegbar gelagert ist.

16. Injektionsgerät nach einem der vorhergehenden Ansprüche, **dadurch gekennzeichnet, dass** das Auslöseglied (18) das Schaltglied (20) durch Abgleiten an dem Schaltglied (20) aus der Koppelposition in die Freigabeposition bewegt.

17. Injektionsgerät nach einem der vorhergehenden Ansprüche, **dadurch gekennzeichnet, dass** wenigstens eines aus Auslöseglied (18) und Schaltglied (20) mit einer Schaltkurve (19) versehen ist, mit der es in einem Koppeleingriff an dem anderen abgleitet, und dass die Schaltkurve (19) zu einer Richtung, in die das Schaltglied (20),aus der Koppelposition in die Freigabeposition bewegt wird, und zu einer Richtung, in die das Auslöseglied (18) in dem Koppeleingriff bei seiner Betätigung bewegt wird, geneigt ist.

18. Injektionsgerät nach einem der vorhergehenden Ansprüche, **dadurch gekennzeichnet, dass** die Antriebseinrichtung (4,10-13) und der Gehäuseabschnitt (1) über wenigstens ein Blockierelement (15; 25) in dem Halteeingriff sind und dass das Schaltglied (20) das Blockierelement (15; 25) gegen eine Elastizitätskraft in dem Halteeingriff hält.

19. Injektionsgerät nach dem vorhergehenden Anspruch, **dadurch gekennzeichnet, dass** das Schaltglied (20) das Blockierelement (15; 25) gegen die Elastizitätskraft in den Halteeingriff drückt.

20. Injektionsgerät nach einem der zwei vorhergehenden Ansprüche, **dadurch gekennzeichnet, dass** das Blockierelement (15; 25) in der Freigabeposition des Schaltglieds (20) von dem Schaltglied (20) wenigstens soweit entlastet ist, dass das Blockierelement (15; 25) aus dem Halteeingriff bewegbar ist, vorzugsweise automatisch aus dem Halteeingriff schnappt.

21. Injektionsgerät nach einem der drei vorhergehenden Ansprüche, **dadurch gekennzeichnet, dass** das Schaltglied (20) wenigstens eine Ausnehmung (24) aufweist, in die das Blockierelement (15; 25) in der Freigabeposition des Schaltglieds (20) schnappt, wodurch sich der Halteeingriff löst.

22. Injektionsgerät nach einem der vier vorhergehenden Ansprüche, **dadurch gekennzeichnet, dass** das Blockierelement (15; 25) sich quer zu der Längsrichtung der Injektionsnadel (5) aus dem Halteeingriff bewegt.

23. Injektionsgerät nach einem der fünf vorhergehenden Ansprüche, **dadurch gekennzeichnet, dass** das Schaltglied (20) einen Hülsenabschnitt aufweist und mit einer Mantelinnenfläche des Hülsenabschnitts das Blockierelement (15; 25) in den Halteeingriff drückt und dass in der Mantelinnenfläche eine Ausnehmung (24) gebildet ist, in die das Blockierelement (15, 25) aus dem Halteeingriff schnappt.

24. Injektionsgerät nach einem der vorhergehenden Ansprüche, **dadurch gekennzeichnet, dass** der Nadelschutz (2a) bei seiner Bewegung in die Rückzugsposition das Schaltglied (20) aus der Verriegelungsposition in die Koppelposition mitnimmt.

25. Injektionsgerät nach dem vorhergehenden Anspruch, **dadurch gekennzeichnet, dass** der Nadelschutz (2a) das Schaltglied (20) durch Druckkontakt mitnimmt.

26. Injektionsgerät nach einem der vorhergehenden Ansprüche, **dadurch gekennzeichnet, dass** das Schaltglied (20) bei der Bewegung aus der Koppelposition in die Freigabeposition von dem Nadelschutz (2a) gelöst ist.

27. Injektionsgerät nach einem der vorhergehenden Ansprüche, **dadurch gekennzeichnet, dass** die Antriebseinrichtung (10-13) einen Einstechantrieb (13) und eine relativ zu dem Gehäuseabschnitt (1) nach distal in eine Vortriebsrichtung (V) bewegbar gelagerte und in dem Halteeingriff gegen eine Antriebskraft des Einstechantriebs (13) blockierte Vortriebsstruktur (12) umfasst und dass nach einem Lösen des Halteeingriffs der Einstechantrieb (13) die Vortriebsstruktur (12) und die Vortriebsstruktur (12) die Injektionsnadel (5) relativ zu dem Nadelschutz (2a) in die Vortriebsrichtung (V) treiben.

28. Injektionsgerät nach dem vorhergehenden Anspruch, **dadurch gekennzeichnet, dass** die Antriebseinrichtung (10-13) ein in dem Reservoir (13) auf das Produkt wirkendes Förderelement (4), eine Ausschüttstruktur (11) und einen Ausschüttantrieb (10) aufweist, der das Förderelement (4) über die Ausschüttstruktur (11) antreibt, und dass die Vortriebsstruktur (12) die Ausschüttstruktur (11) aufnimmt und in die Vortriebsrichtung (V) führt, wobei der Ausschüttantrieb (10) von dem Einstechantrieb oder ein anderen Antrieb gebildet wird.

## Claims

1. An autoinjector comprising an elongate housing in which a container (3) for an active substance is arranged, wherein the housing comprises at least two housing portions (1, 2) movable relative to each other, a drive element (12) which can be biased in the injection direction by the force of at least one spring (7), a release element (20) movable from a locking position in which the drive element (12) is secured in a biased position into a release position in which the drive element (12) is released, and a triggering element (18),
**characterised in that** the release element (20) is movable by a relative movement of the housing portions (1, 2) from its locking position into an intermediate position in which the drive element (12) is secured and that the triggering element (18) is capable of moving the release element (20) from the intermediate position into the release position.

2. An autoinjector according to claim 1 **characterised in that** the triggering element (18) is movable in a plane transversely relative to the longitudinal direction of the autoinjector and the release element (20) is movable in the longitudinal direction of the autoinjector.

3. An autoinjector according to one of the preceding claims **characterised in that** the triggering element (18) has a guide device (19, 21, 22) for guiding the release element (21) in the movement from the intermediate position into the release position.

4. An autoinjector according to one of the preceding claims **characterised in that** the release element (20) co-operates with a securing device (15) for securing the drive element (12) in a biased position.

5. An autoinjector according to one of the preceding claims **characterised in that** the securing device (15) for unlocking is movable in a plane transversely relative to the longitudinal direction of the autoinjector.

6. An autoinjector according to claim 1 **characterised in that** the triggering element (18) is movable in the longitudinal direction of the autoinjector and the release element (20) is movable in a plane transversely relative to the longitudinal direction of the autoinjector.

7. An autoinjector according to one of the preceding claims **characterised in that** the release element (20) in its locking position prevents the triggering element (18) from moving.

8. An autoinjector according to one of the preceding claims **characterised in that** the release element (20) has a keyhole-shaped opening (121) and provided on the drive element is an annular groove (115) into which the narrow part of that keyhole-shaped opening (121) engages in the locking position of the release element (20).

9. An autoinjector according to claim 1 **characterised in that** the release element (20) is so guided that it performs a rotary movement from the locking position into the release position.

10. An autoinjector according to claim 9 **characterised in that** the release element (20) has a non-circular opening (121) and that provided on the drive element is a head of a non-circular cross-section, which passes through the opening only in the release position of the release element.

11. A triggerable injection device including:
a) a housing portion (1),
b) a reservoir (3) for an injectable product,
c) an injection needle (5) with a needle tip at a distal end,
d) a needle protection (2a) movable relative to the housing portion (1) and the injection needle (5) from a protection position in which it surrounds the injection needle (5) to beyond the needle tip in a proximal direction into a retracted position,
e) a drive device (4, 10-13) for an injection, which is locked in a releasable holding engagement to prevent a drive movement,
f) a switching member (20) so coupled to the needle protection (2a) that the movement of the needle protection (2a) in the proximal direction causes a movement of the switching member (20) from a locking position into a coupling position, and
g) an actuatable triggering member (18) for triggering of the drive device (4, 10-13), which upon actuation thereof is coupled to the switching member (20) when the switching member (20) occupies the coupling position and causes a movement of the switching member (20) out of the coupling position into a release position,
h) wherein the holding engagement of the drive device (4, 10-13) is released only in the release position of the switching member (20).

12. An injection device according to the preceding claim **characterised in that** the movement of the switching member (20) from the locking position into the release position is in the same direction.

13. An injection device according to one of the preceding claims **characterised in that** the switching member (20) is moved out of the locking position into the release position in the proximal direction.

14. An injection device according to one of the preceding claims **characterised in that** the switching member (20) is axially linearly guided from the locking position into the release position.

15. An injection device according to one of the preceding claims **characterised in that** the triggering member (18) is mounted movably transversely relative to a direction in which the switching member (20) is moved into the release position.

16. An injection device according to one of the preceding claims **characterised in that** the triggering member (18) moves the switching member (20) from the coupling position into the release position by sliding against the switching member (20).

17. An injection device according to one of the preceding claims **characterised in that** at least one of the triggering member (18) and the switching member (20) is provided with a switching cam (19) with which it slides in a coupling engagement against the other, and the switching cam (19) is inclined relative to a direction in which the switching member (20) is moved from the coupling position into the release position and relative to a direction in which the triggering member (18) is moved in the coupling engagement upon actuation thereof.

18. An injection device according to one of the preceding claims **characterised in that** the drive device (4, 10-13) and the housing portion (1) are in holding engagement by way of at least one blocking element (15; 25) and that the switching member (20) holds the blocking element (15; 25) in the holding engagement against an elasticity force.

19. An injection device according to the preceding claim **characterised in that** the switching member (20) presses the blocking element (15; 25) into the holding engagement against the elasticity force.

20. An injection device according to one of the two preceding claims **characterised in that** in the release position of the switching member (20) the blocking element (15; 25) is relieved of the load of the switching member (20) at least to such an extent that the blocking element (15; 25) is movable out of the holding engagement and preferably automatically snaps out of the holding engagement.

21. An injection device according to one of the three preceding claims **characterised in that** the switching member (20) has at least one recess (24) into which the blocking element (15; 25) snaps in the release position of the switching member (20), whereby the holding engagement is released.

22. An injection device according to one of the four preceding claims **characterised in that** the blocking element (15; 25) is moved transversely relative to the longitudinal direction of the injection needle (5) out of the holding engagement.

23. An injection device according to one of the five preceding claims **characterised in that** the switching member (20) has a sleeve portion and with an inside peripheral surface of the sleeve portion presses the blocking element (15; 25) into the holding engagement and that formed in the inside peripheral surface is a recess (24) into which the blocking element (15, 25) snaps out of the holding engagement.

24. An injection device according to one of the preceding claims **characterised in that** in its movement into the retracted position the needle protection (2a) entrains the switching member (20) out of the locking position into the coupling position.

25. An injection device according to the preceding claim **characterised in that** the needle protection (2a) entrains the switching member (20) by pressure contact.

26. An injection device according to one of the preceding claims **characterised in that** the switching member (20) is released from the needle protection (2a) in the movement out of the coupling position into the release position.

27. An injection device according to one of the preceding claims **characterised in that** the drive device (10-13) has an injection drive (13) and a forward drive structure (12) which is mounted movably distally in a forward drive direction (V) relative to the housing portion (1) and is blocked in the holding engagement against a drive force of the injection drive (13) and that after release of the holding engagement the injection drive (13) drives the forward drive structure (12) and the forward drive structure (12) drives the injection needle (5) relative to the needle protection (2a) in the forward drive direction (V).

28. An injection device according to the preceding claim **characterised in that** the drive device (10-13) has a conveyor element (4) which acts on the product in the reservoir (13), a dispensing structure (11) and a dispensing drive (10) which drives the conveyor element (4) by way of the dispensing structure (11), and that the forward drive structure (12) receives the dispensing structure (11) and guides it in the forward drive direction (V), wherein the dispensing drive (10) is formed by the injection drive or another drive.

## Revendications

1. Auto-injecteur avec un boîtier allongé, dans lequel est agencé un récipient (3) pour une substance active, le boîtier se composant d'au moins deux parties de boîtier (1, 2) qui sont mobiles l'une par rapport à l'autre, avec un élément d'entraînement (12) qui peut être précontraint dans le sens d'injection par la force d'au moins un ressort (7), avec un élément de libération (20) qui peut se déplacer d'une position de blocage dans laquelle l'élément d'entraînement (12) est bloqué dans une position précontrainte, à une position de libération dans laquelle l'élément d'entraînement (12) est débloqué et avec un élément de déclenchement (18),
**caractérisé en ce que** l'élément de libération (20) peut être déplacé par un mouvement relatif des parties du boîtier (1, 2) de sa position de blocage à une position intermédiaire, dans laquelle l'élément d'entraînement (12) est bloqué, et **en ce que** l'élément de déclenchement (18) est apte à déplacer l'élément de libération (20) de la position intermédiaire à la position de libération.

2. Auto-injecteur selon la revendication 1, **caractérisé en ce que** l'élément de déclenchement (18) peut être déplacé dans un plan transversal au sens longitudinal de l'auto-injecteur et l'élément de libération (20) peut être déplacé dans le sens longitudinal de l'auto-injecteur.

3. Auto-injecteur selon l'une quelconque des revendications précédentes, **caractérisé en ce que** l'élément de déclenchement (18) présente un dispositif de guidage (19, 21, 22) servant au guidage de l'élément de libération (20) lors du déplacement de la position intermédiaire à la position de libération.

4. Auto-injecteur selon l'une quelconque des revendications précédentes, **caractérisé en ce que** l'élément de libération (20) coopère avec un dispositif de blocage (15) pour le blocage de l'élément d'entraînement (12) dans une position précontrainte.

5. Auto-injecteur selon l'une quelconque des revendications précédentes, **caractérisé en ce que** le dispositif de blocage (15) peut être déplacé pour le déblocage dans un plan transversal au sens longitudinal de l'auto-injecteur.

6. Auto-injecteur selon la revendication 1, **caractérisé en ce que** l'élément de déclenchement (18) est mobile dans le sens longitudinal de l'auto-injecteur et l'élément de libération (20) est mobile dans un plan transversal au sens longitudinal de l'auto-injecteur.

7. Auto-injecteur selon l'une quelconque des revendications précédentes, **caractérisé en ce que** l'élément de libération (20) dans sa position de blocage empêche le déplacement de l'élément de déclenchement (18).

8. Auto-injecteur selon l'une quelconque des revendications précédentes, **caractérisé en ce que** l'élément de libération (20) a une ouverture en forme de trou de serrure (121), et **en ce qu'**il y a une rainure annulaire (115) sur l'élément d'entraînement, dans laquelle la partie étroite de cette ouverture en forme de trou de serrure (121) s'encliquète dans la position de blocage de l'élément de libération (20).

9. Auto-injecteur selon la revendication 1, **caractérisé en ce que** l'élément de libération (20) est guidé de telle sorte qu'il réalise un mouvement de rotation de la position de blocage à la position de libération.

10. Auto-injecteur selon la revendication 9, **caractérisé en ce que** l'élément de libération (20) a une ouverture non circulaire (121) et **en ce qu'**il y a une tête avec une section transversale non circulaire sur l'élément d'entraînement, laquelle passe seulement par l'ouverture lorsque l'élément de libération est en position de libération.

11. Dispositif d'injection déclenchable comprenant :
a) une section de boîtier (1)
b) un réservoir (3) pour un produit injectable
c) une aiguille d'injection (5) avec une pointe d'aiguille à une extrémité distale
d) un protège-aiguille (2a) qui peut se déplacer par rapport à la partie de boîtier (1) et à l'aiguille d'injection (5) d'une position de protection, dans laquelle il entoure l'aiguille d'injection (5) jusqu'au-dessus de la pointe de l'aiguille, vers proximal jusque dans une position de rétraction,
e) un dispositif d'entraînement (4, 10 - 13) pour une injection, qui est bloqué contre un mouvement d'entraînement dans une prise de retenue libérable,
f) un organe de commande (20), qui est couplé avec le protège-aiguille (2a) de telle sorte que le déplacement du protège-aiguille (2a) vers proximal induit un déplacement de l'organe de commande (20) de la position de verrouillage à une position de couplage,
g) et un organe de déclenchement (18) actionnable pour un déclenchement du dispositif d'entraînement (4, 10 - 13) qui est couplé lors de son actionnement avec l'organe de commande (20), lorsque l'organe de commande (20) adopte la position de couplage, et induit un déplacement de l'organe de commande (20) de la position de couplage à une position de libération,
h) la prise de retenue du dispositif d'entraînement (4, 10 - 13) ne se déclenchant que lorsque l'organe de commande (20) est en position de libération.

12. Dispositif d'injection selon la revendication précédente, **caractérisé en ce que** le déplacement de l'organe de commande (20) de la position de verrouillage jusqu'à la position de libération est dirigé le même sens.

13. Dispositif d'injection selon l'une quelconque des revendications précédentes **caractérisé en ce que** l'organe de commande (20) est déplacé de la position de verrouillage jusqu'à la position de libération dans le sens proximal.

14. Dispositif d'injection selon l'une quelconque des revendications précédentes, **caractérisé en ce que** l'organe de commande (20) est guidé de la position de verrouillage jusqu'à la position de libération de manière axialement linéaire.

15. Dispositif d'injection selon l'une quelconque des revendications précédentes, **caractérisé en ce que** l'organe de déclenchement (18) est logé de manière mobile transversalement à une direction dans laquelle l'organe de commande (20) est déplacé dans la position de libération.

16. Dispositif d'injection selon l'une quelconque des revendications précédentes, **caractérisé en ce que** l'organe de déclenchement (18) déplace l'organe de commande (20) par glissement sur l'organe de commande (20) de la position de couplage à la position de libération.

17. Dispositif d'injection selon l'une quelconque des revendications précédentes, **caractérisé en ce qu'**au moins un parmi l'organe de déclenchement (18) et l'organe de commande (20) est doté d'une came de commande (19), avec laquelle il glisse sur l'autre dans une prise de couplage, et **en ce que** la came de commande (19) est inclinée dans une direction dans laquelle l'organe de commande (20) est déplacé de la position de couplage à la position de libération, et dans une direction dans laquelle l'organe de déclenchement (18) est déplacé dans la prise de couplage lors de son actionnement.

18. Dispositif d'injection selon l'une quelconque des revendications précédentes, **caractérisé en ce que** le dispositif d'entraînement (4, 10 - 13) et la partie de boîtier (1) sont en prise de retenue par le biais d'au moins un élément de blocage (15 ; 25), et **en ce que** l'organe de commande (20) retient l'élément de blocage (15 ; 25) contre une force d'élasticité dans la prise de retenue.

19. Dispositif d'injection selon la revendication précédente, **caractérisé en ce que** l'organe de commande (20) pousse l'élément de blocage (15 ; 25) contre la force d'élasticité dans la prise de retenue.

20. Dispositif d'injection selon l'une quelconque des deux revendications précédentes, **caractérisé en ce que** l'élément de blocage (15 ; 25) est déchargé dans la position de libération de l'organe de commande (20) par l'organe de commande (20) au moins jusqu'à ce que l'élément de blocage (15 ; 25) puisse être déplacé hors de la prise de retenue, de préférence sort automatiquement de la prise de retenue.

21. Dispositif d'injection selon l'une quelconque des trois revendications précédentes, **caractérisé en ce que** l'organe de commande (20) présente au moins un évidement (24), dans lequel s'enclenche l'élément de blocage (15 ; 25) dans la position de libération de l'organe de commande (20), ce qui libère la prise de retenue.

22. Dispositif d'injection selon l'une quelconque des quatre revendications précédentes, **caractérisé en ce que** l'élément de blocage (15 ; 25) se déplace transversalement au sens longitudinal de l'aiguille d'injection (5) hors de la prise de retenue.

23. Dispositif d'injection selon l'une quelconque des cinq revendications précédentes, **caractérisé en ce que** l'organe de commande (20) présente une section de douille et pousse l'élément de blocage (15 ; 25) dans la prise de retenue avec une surface intérieure de l'enveloppe de la section de douille, et **en ce qu'**un évidement (24), dans lequel l'élément de blocage (15, 25) sort de la prise de retenue, est formé dans la surface intérieure de l'enveloppe.

24. Dispositif d'injection selon l'une quelconque des revendications précédentes, **caractérisé en ce que**, lors de son déplacement dans la position de rétraction, le protège-aiguille (2a) entraîne l'organe de commande (20) de la position de verrouillage à la position de couplage.

25. Dispositif d'injection selon la revendication précédente, **caractérisé en ce que** le protège-aiguille (2a) entraîne l'organe de commande (20) par contact de pression.

26. Dispositif d'injection selon l'une quelconque des revendications précédentes, **caractérisé en ce que**, lors du déplacement de la position de couplage à la position de libération, l'organe de commande (20) est libéré du protège-aiguille (2a).

27. Dispositif d'injection selon l'une quelconque des revendications précédentes, **caractérisé en ce que** le dispositif d'entraînement (10 - 13) comprend un entraînement pour piqûre (13) et une structure d'avancement (12) logée de manière mobile par rapport à la partie de boîtier (1) vers distal dans un sens d'avancement (V) et bloquée dans la prise de retenue contre une force d'entraînement de l'entraînement pour piqûre (13), et **en ce que** après libération de la prise de retenue, l'entraînement pour piqûre (13) entraîne la structure d'avancement (12) dans le sens d'avancement (V) et la structure d'avancement (12) entraîne l'aiguille d'injection (5) par rapport au protège-aiguille (2a) dans le sens d'avancement (V).

28. Dispositif d'injection selon la revendication précédente, **caractérisé en ce que** le dispositif d'entraînement (10 - 13) présente un élément de transport (4) agissant sur le produit dans le réservoir (13), une structure de déversement (11) et un entraînement de déversement (10), qui entraîne l'élément de transport (4) par le biais de la structure de déversement (11), et **en ce que** la structure d'avancement (12) reçoit la structure de déversement (11) et conduit dans le sens d'avancement (V), à l'occasion de quoi l'entraînement de déversement (10) de l'entraînement pour piqûre ou un d'autre entraînement, est formé.
